# EUROPEAN PATENT APPLICATION

(11) **EP 3 845 148 A1**
(43) Date of publication of application: **07.07.2021**
(21) Application number: 19865783.5
(22) Date of filing: 26.09.2019
(51) Int. Cl.: A61B 17/11, A61L 17/06

(54) **ADHESION PROMOTION DEVICE AND MEDICAL DEVICE**

(30) Priority: 27.09.2018 JP 2018181774
(71) Applicant: TERUMO Kabushiki Kaisha, Tokyo 151-0072 (JP)
(72) Inventor: ARAMAKI, Naoki, Ashigarakami-gun, Kanagawa 259-0151 (JP); KAI, Miho, Ashigarakami-gun, Kanagawa 259-0151 (JP); HATA, Mayu, Ashigarakami-gun, Kanagawa 259-0151 (JP)
(74) Representative: Casalonga
(86) International application number: PCT/JP2019/038038
(87) International publication number: WO 2020/067380

(57) **Abstract**

[Problem]

There are provided an adhesion promotion device and a medical device which are capable of effectively reducing risk factors of an anastomotic leakage.

[Means for Resolution]

An adhesion promotion device 100 has a main body portion 20 formed of a biodegradable sheet having a plurality of through-holes 25 and promoting adhesion of biological tissues, and an assistance unit 40 provided in the main body portion and assisting the main body portion to hold a biological organ serving as a joint object.

## Description

### Technical Field

The present invention relates to an adhesion promotion device and a medical device.

### Background Art

In a medical field, a medical procedure (for example, anastomosis for a digestive tract) of joining biological organs to each other by performing a surgical operation is known. In a case where the medical procedure as described above is performed, as a prognosis determinant after surgery, a fact is important that there is no delay in adhesion in a joint portion joined between the biological organs.

In the medical procedure of joining the biological organs, various methods and various medical instruments are used. For example, a method of suturing the biological organs by using a biodegradable suture, or a method of using a mechanical anastomosis device (refer to PTL 1) for suturing the biological organs by using a stapler has been proposed. In particular, in a case where anastomosis is performed using the mechanical anastomosis device, compared to a method of using the suture, a joining force between the biological organs can be improved in the joint portion. Accordingly, risk factors of an anastomotic leakage can be reduced.

### Citation List

### Patent Literature

PTL 1: JP-T-2007-505708

### Summary of Invention

### Technical Problem

However, a progress degree of the adhesion in the joint portion depends on a state of biological tissues in a joint object site (joint target site) of a patient. Therefore, for example, even in a case where the anastomosis device as disclosed in PTL 1 is used, depending on the state of the biological tissues of the patient, there is a possibility that the risk factors of the anastomotic leakage cannot be sufficiently reduced.

Therefore, the present invention aims to provide an adhesion promotion device and a medical device which are capable of effectively reducing risk factors of an anastomotic leakage. Solution to Problem

According to the present invention, there is provided an adhesion promotion device including a main body portion formed of a biodegradable sheet having a plurality of through-holes and promoting adhesion of biological tissues, and an assistance unit provided in the main body portion and assisting the main body portion to hold a biological organ serving as a joint object.

### Advantageous Effects of Invention

According to the adhesion promotion device of the present invention, the main body portion is pinched between the biological organs serving as the joint objects. In this manner, it is possible to promote adhesion of the biological tissues of the biological organs. In addition, while an operator performs a medical procedure, the operator uses the assistance unit provided in the main body portion to assist the main body portion to hold the biological organ serving as the joint object. In this manner, it is possible to prevent a possibility that the main body portion may be misaligned from the biological organ or may be distorted and deformed. Therefore, the operator can effectively reduce risk factors of an anastomotic leakage of the biological organs.

### Brief Description of Drawings

[Fig. 1] Fig. 1 is a perspective view illustrating an adhesion promotion device according to a first embodiment of the present invention.
[Fig. 2] Fig. 2 is an enlarged view illustrating a portion of a cross section of a main body portion taken along arrow line 2A-2A illustrated in Fig. 1.
[Fig. 3] Fig. 3 is a flowchart illustrating each procedure of a treatment method of using the adhesion promotion device.
[Fig. 4] Fig. 4 is a flowchart illustrating a procedure of a treatment method (large intestine anastomosis) according to an embodiment.
[Fig. 5] Fig. 5 is a cross-sectional view schematically illustrating an example of a treatment procedure using the adhesion promotion device according to the first embodiment.
[Fig. 6] Fig. 6 is a cross-sectional view schematically illustrating an example of the treatment procedure using the adhesion promotion device according to the first embodiment.
[Fig. 7] Fig. 7 is a cross-sectional view schematically illustrating an example of the treatment procedure using the adhesion promotion device according to the first embodiment.
[Fig. 8] Fig. 8 is a perspective view illustrating an adhesion promotion device according to a modification example of the first embodiment.
[Fig. 9] Fig. 9 is a perspective view illustrating an adhesion promotion device according to a second embodiment of the present invention.
[Fig. 10] Fig. 10 is a cross-sectional view schematically illustrating an example of a treatment procedure using the adhesion promotion device according to the second embodiment.
[Fig. 11] Fig. 11 is a perspective view illustrating an adhesion promotion device according to Modification Example 1 of the second embodiment.
[Fig. 12] Fig. 12 is a perspective view illustrating an adhesion promotion device according to Modification Example 2 of the second embodiment.
[Fig. 13] Fig. 13 is an enlarged view illustrating a portion of the adhesion promotion device according to Modification Example 2 of the second embodiment.
[Fig. 14] Fig. 14 is a plan view illustrating an adhesion promotion device according to Modification Example 3 of the second embodiment.
[Fig. 15] Fig. 15 is a cross-sectional view schematically illustrating an example of a treatment procedure using the adhesion promotion device according to Modification Example 3 of the second embodiment.
[Fig. 16] Fig. 16 is a plan view illustrating an adhesion promotion device according to Modification Example 4 of the second embodiment.
[Fig. 17] Fig. 17 is a perspective view illustrating an assistance device according to the present invention.
[Fig. 18] Fig. 18 is a cross-sectional view schematically illustrating an example of a treatment procedure using the assistance device.
[Fig. 19] Fig. 19 is a perspective view illustrating a modification example of the assistance device.
[Fig. 20] Fig. 20 is a perspective view illustrating another modification example of the assistance device.

### Description of Embodiments

### (First Embodiment)

Hereinafter, embodiments of the present invention will be described with reference to the accompanying drawings.

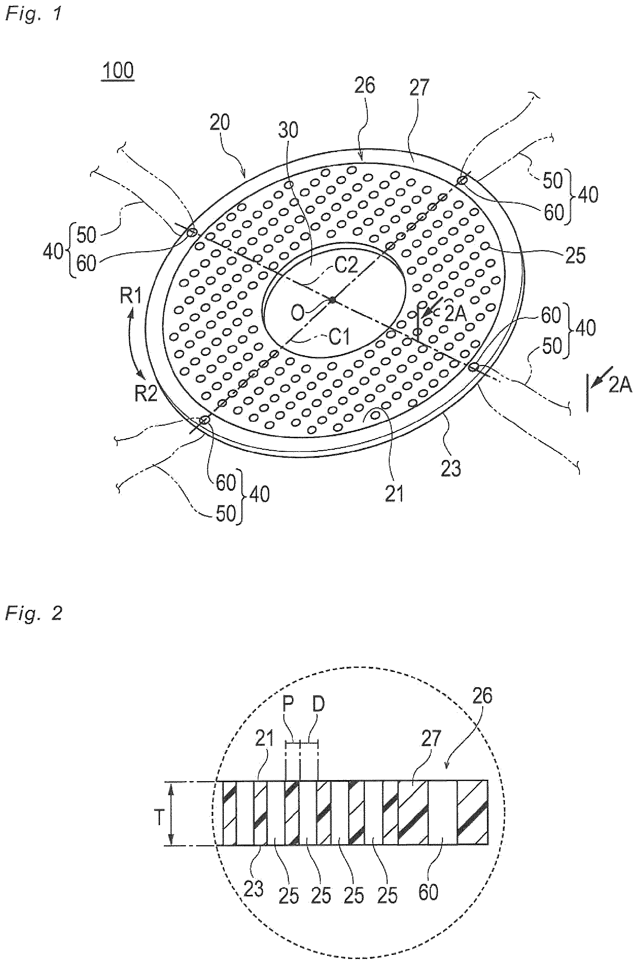

Fig. 1 illustrates an adhesion promotion device 100 according to a first embodiment. Fig. 2 illustrates a cross-sectional view of a main body portion 20 taken along arrow line 2A-2A illustrated in Fig. 1.

### <Adhesion Promotion Device 100>

As illustrated in Fig. 1, the adhesion promotion device 100 includes the main body portion 20 formed of a biodegradable sheet having a plurality of through-holes 25 and promoting adhesion of biological tissues, and an assistance unit 40 provided in the main body portion 20 and assisting the main body portion 20 to hold a biological organ serving as a joint object.

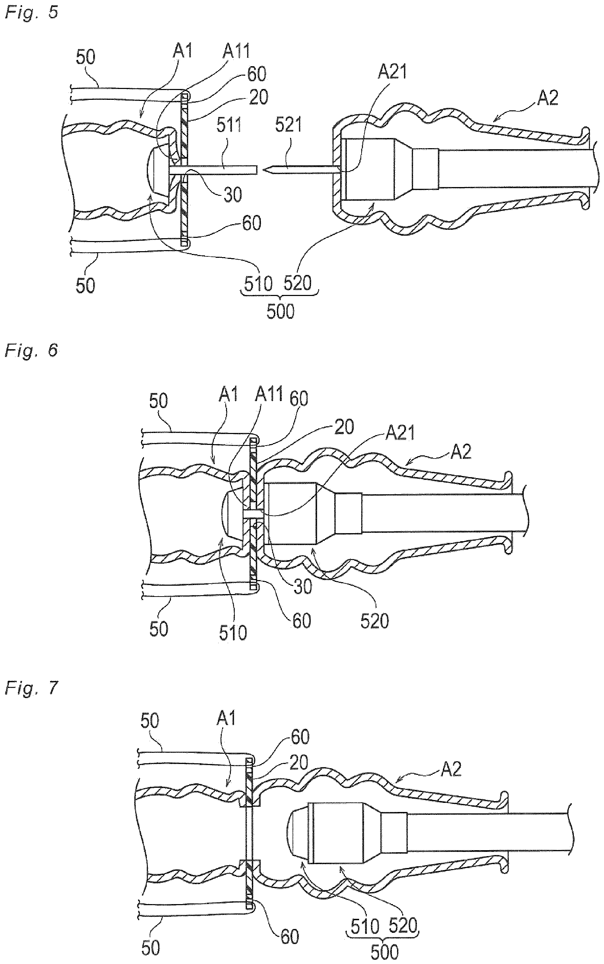

As illustrated in Figs. 5 to 7, the adhesion promotion device 100 is applicable to a medical procedure (for example, anastomosis of a digestive tract) for joining predetermined biological organs to each other. As will be described later, in the description herein, large intestine anastomosis will be described as an example of the medical procedure using the adhesion promotion device 100.

### <Main Body Portion 20>

As illustrated in Fig. 1, the main body portion 20 is configured to include a sheet-like member. For example, the main body portion 20 can be formed of the biodegradable sheet.

As illustrated in Fig. 1, the respective through-holes 25 formed in the main body portion 20 are regularly and cyclically provided in a plane direction of the main body portion 20. However, the respective through-holes 25 may be randomly provided in each portion in the plane direction of the main body portion 20.

As illustrated in Fig. 2, the respective through-holes 25 extend substantially vertically between a front surface 21 and a rear surface 23 along a thickness direction (upward-downward direction in Fig. 2) of the main body portion 20. The respective through-holes 25 may be bent or curved in a zigzag shape between the front surface 21 and the rear surface 23 in a cross section along the thickness direction of the main body portion 20.

The respective through-holes 25 have a substantially circular planar shape (shape when the front surface 21 of the main body portion 20 or the rear surface 23 of the main body portion 20 is viewed in a plan view). However, the planar shape of the respective through-holes 25 is not particularly limited, and for example, may be an elliptical shape or a polygonal shape (rectangular shape or triangular shape). In addition, each of the through-holes 25 may have the different planar shape or the different cross-sectional shape.

The main body portion 20 has a substantially circular planar shape. However, the planar shape of the main body portion 20 is not particularly limited, and for example, may be an elliptical shape or a polygonal shape (rectangular shape or triangular shape).

A thickness (dimension T illustrated in Fig. 2) of the main body portion 20 is not particularly limited, but is preferably 0.05 mm to 0.3 mm, and is more preferably 0.1 mm to 0.2 mm. In a case where the thickness of the main body portion 20 is 0.05 mm or thicker (particularly in a case of 0.1 mm or thicker), the main body portion 20 can be strong to such an extent that the main body portion 20 is not damaged when the adhesion promotion device 10 is handled. On the other hand, in a case where the thickness of the main body portion 20 is 0.3 mm or thinner (particularly in a case of 0.2 mm or thinner), the main body portion 20 can be sufficiently flexible in following the biological tissue when the main body portion 20 is in close contact with the biological tissue to which the main body portion 20 is applied.

In the main body portion 20, a ratio value of a hole diameter D (distance D illustrated in Fig. 2) of the through-hole 25 with respect to a pitch P (distance P illustrated in Fig. 2, and a distance between the through-holes 25 adjacent to each other) of the through-holes 25 is preferably 0.25 or greater and smaller than 40. In a case where a planar shape of the through-hole 25 is a perfect circle, the hole diameter D of the through-hole 25 is equal to a diameter of the perfect circle. On the other hand, in a case where the planar shape of the through-hole 25 is not the perfect circle, the diameter (equivalent circle diameter) of the perfect circle having an area the same as an area of an opening portion (portion facing the front surface 21 or the rear surface 23 in the through-hole 25) of the through-hole 25 can be set as the hole diameter D of the through-hole 25.

Since the main body portion 20 has the plurality of through-holes 25, the main body portion 20 has a plurality of values of the hole diameters D corresponding to the respective through-holes 25. Therefore, in the present embodiment, in calculating the above-described ratio value, an arithmetic average value of two or more values of the hole diameter D corresponding to each of the plurality of through-holes 25 is used as a representative value of the hole diameter D. On the other hand, the pitch P of the plurality of through-holes 25 means a shortest distance between the opening portions of the two through-holes 25. However, with regard to the value of the pitch P, there are a plurality of values of the pitch P corresponding to a combination of the through-holes 25 adjacent to each other. Therefore, according to the present embodiment, in calculating the above-described ratio value, the arithmetic average value of two or more values of the pitch P corresponding to each combination of the through-holes 25 adjacent to each other is used as a representative value of the pitch P.

The pitch P of the above-described through-holes 25, the hole diameter D, and the ratio of the hole diameter D to the pitch P are merely examples, and the present invention is not limited thereto.

The main body portion 20 can be formed of a biodegradable material. A configuration material of the main body portion 20 is not particularly limited. For example, a biodegradable resin may be used. As the biodegradable resin, for example, it is possible to use a known biodegradable (co)polymer such as those disclosed in JP-T-2011-528275, JP-T-2008-514719, Pamphlet of International Publication No. 2008-1952, and JP-T-2004-509205. Specifically, the biodegradable resin includes (1) a polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose; or (2) copolymer configured to include one or more monomers configuring the above-described materials (1). That is, it is preferable that the biodegradable sheet includes the polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose, and at least one biodegradable resin selected from a group formed of the copolymer configured to include one or more monomers configuring the polymer.

A manufacturing method of the main body portion 20 is not particularly limited. For example, the manufacturing method includes a method of preparing a fiber formed of the above-described biodegradable resin and manufacturing a mesh-shaped sheet by using the fiber. A method of preparing the fiber formed of the biodegradable resin is not particularly limited. For example, the method includes an electrospinning method (electric field spinning method and electrostatic spinning method) or a melt blowing method. As the method for the main body portion 20, only one of the above-described methods may be selected and used. Alternatively, two or more methods may be used in appropriate combination with each other. As still another example of the manufacturing method of the main body portion 20, a fiber formed of the above-described biodegradable resin may be spun in accordance with a usual method, and the obtained fiber may be knitted into a mesh shape to manufacture the biodegradable sheet according to the present invention.

The main body portion 20 causes a biological reaction by using the configuration materials such as the biodegradable resin configuring the main body portion 20. Due to this action, the main body portion 20 induces expression of biological components such as fibrin. The biological components induced in this way can promote adhesion by being accumulated to penetrate the through-holes 25 of the main body portion 20. Therefore, the main body portion 20 of the adhesion promotion device 100 is disposed between the biological organs serving as the joint object, thereby promoting the adhesion by using the above-described mechanism.

In the main body portion 20, a peripheral edge portion (outermost peripheral portion) 26 of the main body portion 20 has a reinforcement portion 27 having rigidity higher than that of the other portion (portion where the through-hole 25 is formed) of the main body portion 20. The reinforcement portion 27 is formed over an entire range of the peripheral edge portion 26 along a circumferential direction (direction indicated by arrows R1-R2 in the drawing) of the main body portion 20. The reinforcement portion 27 may be formed only in a portion of the peripheral edge portion 26.

A method for forming the reinforcement portion 27 is not particularly limited. However, for example, the reinforcement portion 27 can be provided by fixing (integrating) a reinforcement member (reinforcement layer) formed separately from the main body portion 20 to the peripheral edge portion 26 on the front surface 21 side of the main body portion 20 and the peripheral edge portion 26 on the rear surface 23 side of the main body portion 20. For example, the reinforcement member can be formed of a biodegradable sheet having no hole portion such as the through-hole 25, or a resin sheet having the rigidity higher than that of the main body portion 20.

The through-hole 25 is not formed in the peripheral edge portion 26 of the biodegradable sheet which is the configuration material of the main body portion 20. In this manner, the reinforcement portion 27 can be provided in the main body portion 20.

In addition, the through-hole 25 may be formed in the peripheral edge portion 26 of the biodegradable sheet which is the configuration material of the main body portion 20. Thereafter, only the peripheral edge portion 26 may be compressed or heated in the thickness direction to crush the through-hole 25. In this manner, a portion where the configuration materials of the biodegradable sheet are densely aggregated may be formed so that the portion is used as the reinforcement portion 27.

As illustrated in Fig. 1, the main body portion 20 has a hole portion 30 formed to have the hole diameter larger than that of the through-hole 25. The hole portion 30 is formed in a range including a central position O (central position in a plan view) of the main body portion 20. The central position O is a rotation center of the main body portion 20 in a case where the main body portion 20 has a rotationally symmetric shape.

The hole portion 30 has a circular planar shape. The hole diameter of the hole portion 30 can be formed to be 5 mm to 25 mm, for example. The planar shape of the hole portion 30 is not particularly limited, and for example, may be an elliptical shape or a polygonal shape (rectangular shape or triangular shape). In addition, a size of the hole portion 30 is not particularly limited.

### <Assistance Unit 40>

As illustrated in Fig. 1, the assistance unit 40 has a pulling unit 50 pulled to apply a pulling force to the main body portion 20, and a connection section 60 enabling the pulling unit 50 to be connected to the main body portion 20.

The pulling unit 50 can be configured to include an elongated member (for example, a narrow string-shaped member or a strip-shaped member having a predetermined width) having a predetermined length and a cross-sectional shape into which the connection section 60 can be inserted. The cross-sectional shape or the length of the pulling unit 50 is not particularly limited.

The connection section 60 is configured to include an insertion hole formed in the main body portion 20 and penetrating the main body portion 20 in the thickness direction. The connection section 60 is formed in the reinforcement portion 27 of the main body portion 20.

As illustrated in Fig. 1, the adhesion promotion device 100 has four connection sections 60 respectively disposed at different locations in the circumferential direction of the main body portion 20, and four pulling units 50 provided corresponding to the respective connection sections 60. The respective connection sections 60 and the respective pulling units 50 configure the assistance unit 40. That is, the adhesion promotion device 100 according to the present embodiment has the four assistance units 40 respectively disposed at different locations in the circumferential direction of the main body portion 20.

In a case where the plurality of assistance units 40 are provided in one main body portion 20 as in the adhesion promotion device 100, it is preferable that at least one set of the assistance units 40 is provided to form a pair at facing locations of the main body portion 20. In the present embodiment, as illustrated in Fig. 1, two sets of the assistance units 40 are provided to form pairs at facing locations of the main body portion 20. The above-described facing locations of the main body portion 20 mean positions facing across the central position O of the main body portion 20 in two virtual lines C1 and C2 orthogonal at the central position O of the main body portion 20, in a case where the main body portion 20 has a rotationally symmetric circular shape as illustrated in Fig. 1.

For example, the pulling unit 50 can be formed of thermoplastic elastomer such as vinyl chloride, polyurethane elastomer, polystyrene elastomer, styrene-ethylene-butylene-styrene copolymer (SEBS), and styrene-ethylene-propylene-styrene copolymer (SEPS), thermoplastic resin such as nylon and PET, rubber, silicone elastomer, fiber material, or metal such as a SUS wire, a copper wire, a titanium wire, and a Ni-Ti alloy wire. However, the pulling unit 50 is not particularly limited as long as the pulling force (tensile force) can be transmitted to the main body portion 20 in a state of being inserted into the connection section (insertion hole) 60.

As described above, the assistance unit 40 according to the present embodiment includes the pulling unit 50 and the connection section 60. However, the assistance unit 40 may be configured to include only the connection section 60, or may be configured to include only the pulling unit 50 as will described in an embodiment (to be described later) . In a case where the pulling unit 50 is not configured to be integral with the main body portion 20 unlike the adhesion promotion device 100 according to the present embodiment, the operator prepares the pulling unit 50 separated from the adhesion promotion device 100 during a medical procedure. The operator can use the adhesion promotion device 100 and the pulling unit 50 in an appropriate combination in accordance with a progress of the medical procedure.

In addition, the number of the assistance units 40 provided in the adhesion promotion device 100 is not particularly limited. For example, one adhesion promotion device 100 may be provided with only one connection section 60, may be provided with one pulling unit 50, or may be provided with one connection section 60 and one pulling unit 50 (one assistance unit 40). However, it is preferable to dispose at least two or more assistance units 40 so that the pulling force can be efficiently transmitted to the main body portion 20, when the pulling force is applied to the main body portion 20 via the assistance unit 40. As a disposition method, it is more preferable that the assistance units 40 are disposed on a diagonal line (positions facing across the central position O of the main body portion 20).

### <Embodiment of Treatment Method (Large Intestine Anastomosis)>

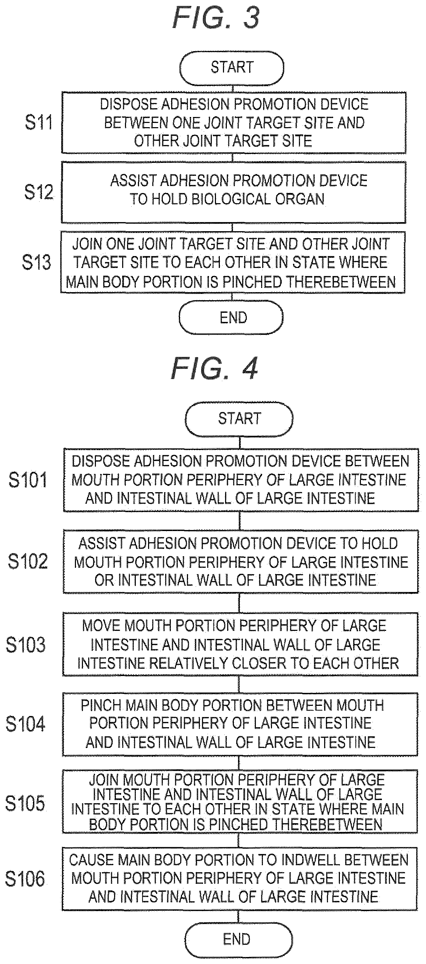

Next, a treatment method to which the adhesion promotion device 100 is applied will be described. Fig. 3 is a flowchart illustrating each procedure of the treatment method to which the adhesion promotion device 100 is applied.

The treatment method (hereinafter, simply referred to as the "treatment method") to which the adhesion promotion device 100 is applied includes disposing the adhesion promotion device including the sheet-like main body portion for promoting the adhesion of the biological tissues between one joint target site and the other joint target site which are joint objects of the biological organ (S11), assisting the adhesion promotion device to hold the biological organ (S12), and joining one joint target site and the other joint target site to each other in a state where at least a portion of the main body portion of the adhesion promotion device is disposed between one joint target site and the other joint target site (S13).

The biological organ and the joint target site of the biological organ to which the treatment method according to the present embodiment is applied are not particularly limited, and can be selected in any desired way. However, in the following description, an example will be described in which the treatment method according to the present embodiment is applied to large intestine anastomosis. In addition, in the following description, detailed description of a known medical procedure, a known medical device, or a known medical instrument will be omitted as appropriate.

Hereinafter, "disposing the adhesion promotion device between the biological organs" in the description herein means at least one of disposing the adhesion promotion device in a state where the adhesion promotion device is in direct or indirect contact with the biological organs", disposing the adhesion promotion device in a state where a spatial gap is formed with the biological organ, and disposing the adhesion promotion device in both the states (for example, disposing the adhesion promotion device in a state where the adhesion promotion device is in contact with one biological organ and the adhesion promotion device is not in contact with the other biological organ). In addition, in the description herein, a "periphery" does not define a strict range (region), and means a predetermined range (region) as long as a treatment purpose (joining the biological organs to each other) can be achieved. In addition, as long as the treatment purpose can be achieved, in the medical procedure described in the respective treatment methods, orders can be appropriately switched therebetween. In addition, in the description herein, "moving the portions relatively closer to each other" means both moving two or more movement objects closer to each other, and moving only one closer to the other one. In addition, "assisting the adhesion promotion device to hold the biological organ" includes at least one of work for causing the adhesion promotion device to hold the biological organ, preparation work for carrying out holding work, and work for maintaining the holding of the biological organ.

### <Embodiment of Treatment Method (Large Intestine Anastomosis)>

Fig. 4 is a flowchart illustrating a procedure of large intestine anastomosis, and Figs. 5 to 7 are views for describing the large intestine anastomosis.

In the treatment method according to the present embodiment, the biological organ serving as the joint object is a large intestine cut due to excision of a cancer tumor. Specifically, the biological organs serving as the joint object are a cut mouth side A1 of the large intestine and a cut anal side A2 of the large intestine. In the following description, a joining procedure will be described in which a mouth portion periphery (one joint target site) on the cut mouth side A1 of the large intestine and a portion (the other joint target site) of an intestinal wall on the cut anal side A2 of the large intestine are joined to each other.

As illustrated in Fig. 4, the treatment method according to the present embodiment includes disposing the adhesion promotion device between the mouth portion periphery of the large intestine and the intestinal wall of the large intestine (S101), assisting the adhesion promotion device to hold the mouth portion periphery of the large intestine or the intestinal wall of the large intestine (S102), moving the mouth portion periphery of the large intestine and the intestinal wall of the large intestine relatively closer to each other (S103), pinching the main body portion of the adhesion promotion device between the mouth portion periphery of the large intestine and the intestinal wall of the large intestine (S104), joining the mouth portion periphery of the large intestine and the intestinal wall of the large intestine to each other in a state where the main body portion of the adhesion promotion device is pinched therebetween (S105), and causing the main body portion of the adhesion promotion device to indwell between the mouth portion periphery of the large intestine and the intestinal wall of the large intestine (S106).

The treatment method according to the present embodiment will be described in detail with reference to Figs. 5 to 7.

As illustrated in Fig. 5, an operator inserts a first engagement instrument 510 of an anastomosis device 500 into the mouth side A1 of the large intestine. The operator disposes a second engagement instrument 520 of the anastomosis device 500 on the anal side A2 of the large intestine. Before disposing the second engagement instrument 520 on the anal side A2 of the large intestine, the operator forms a through-hole A21 for inserting the second engagement instrument 520 of the anastomosis device 500 into the anal side A2 of the large intestine. A timing for forming the through-hole A21 is not particularly limited as long as the through-hole A21 is formed before the second engagement instrument 520 is disposed.

For example, as the anastomosis device 500, for example, a known device used for the large intestine anastomosis can be used. The anastomosis device 500 is configured as follows. As the first engagement instrument 510 and the second engagement instrument 520 engage with each other, the biological tissue disposed between the first engagement instrument 510 and the second engagement instrument 520 is excised, and a periphery of the excised biological tissue is sutured into a circumferential shape by using a stapler. For example, the first engagement instrument 510 is an instrument including a cylindrical engagement target portion 511. For example, the second engagement instrument 520 is an instrument including an engagement pin 521 to engage with and to be inserted into the engagement target portion 511 of the first engagement instrument 510.

The operator inserts the engagement target portion 511 of the first engagement instrument 510 into the mouth side A1 of the large intestine, and performs purse-string suture on the engagement target portion 511 in a protruding state, thereby forming a suture portion A11. An outer surface of the suture portion A11 has an uneven shape due to the suture.

Next, as illustrated in Fig. 5, the operator disposes the main body portion 20 of the adhesion promotion device 100 between the mouth side A1 of the large intestine and the anal side A2 of the large intestine. In the present embodiment, the hole portion 30 is formed in the main body portion 20. Therefore, when the main body portion 20 is disposed, the operator can dispose the main body portion 20 in the engagement target portion 511 included in the first engagement instrument 510 by hooking the main body portion 20 on the engagement target portion 511 through the hole portion 30. The operator brings the main body portion 20 into contact with a peripheral portion where the suture portion A11 is formed on the mouth side A1 of the large intestine. The operator may dispose the main body portion 20 on the anal side A2 of the large intestine by causing the engagement pin 521 included in the second engagement instrument 520 to pass through the hole portion 30.

Next, in order to prevent a possibility that the main body portion 20 may be misaligned from the mouth side A1 of the large intestine or may be distorted and deformed, the operator carries out work for assisting the main body portion 20 to hold the mouth side A1 of the large intestine. Specifically, the operator pulls the pulling unit 50 of the assistance unit 40 to one end of the large intestine (side opposite to a side facing the anal side A2 of the large intestine), thereby pressing the main body portion 20 against the mouth side A1 of the large intestine. Therefore, the main body portion 20 can be firmly held for the suture portion A11 and a peripheral portion thereof which form an uneven surface shape. In this case, in order for the main body portion 20 to more stably hold the mouth side A1 of the large intestine, it is preferable that the operator simultaneously pulls a set of the pulling units 50 disposed at facing locations of the main body portion 20. In addition, the operator can maintain a state where the pulling force is applied to the main body portion 20 by pinching the pulling unit 50 with a medical instrument such as intestinal forceps or a clip.

A timing for connecting the pulling unit 50 to the main body portion 20 is not particularly limited. For example, the timing may be after the main body portion 20 is disposed between the mouth side A1 of the large intestine and the anal side A2 of the large intestine, or may be before the adhesion promotion device 100 is introduced into a living body.

In addition, in a case where the operator disposes the main body portion 20 on the anal side A2 of the large intestine, the operator can pull the pulling unit 50 of the assistance unit 40 to the other end of the large intestine (side opposite to a side facing the mouth side A1 of the large intestine). In this manner, the main body portion 20 can be held for the anal side A2 of the large intestine.

Next, while the operator applies the pulling force to the pulling unit 50 and maintains a state where the main body portion 20 is held for the mouth side A1 of the large intestine, as illustrated in Fig. 6, the operator moves the first engagement instrument 510 and the second engagement instrument relatively closer to each other, and causes both of these to engage with each other. The operator pinches the mouth portion periphery on the mouth side A1 of the large intestine, the main body portion 20 of the adhesion promotion device 100, and the periphery of the through-hole A21 formed on the intestinal wall on the anal side A2 of the large intestine, between the first engagement instrument 510 and the second engagement instrument 520. The operator operates the anastomosis device 500. In this manner, while excising a portion on the mouth side A1 of the large intestine, a portion of the main body portion 20 of the adhesion promotion device 100, and a portion on the anal side A2 of the large intestine which are pinched between the first engagement instrument 510 and the second engagement instrument 520, the operator joins the peripheries of the excised sites to each other by using a stapler (not illustrated).

Next, as illustrated in Fig. 7, the operator removes the anastomosis device 500 to the outside of the living body from the anal side A2 of the large intestine via an anus, for example. In addition, the operator causes a portion of the main body portion 20 of the adhesion promotion device 100 pinched between the mouth portion periphery on the mouth side A1 of the large intestine and the intestinal wall on the anal side A2 of the large intestine to indwell. After joining the mouth side A1 of the large intestine and the anal side A2 of the large intestine to each other, the operator can remove the pulling unit 50 connected to the main body portion 20 to the outside of the living body. When the operator removes the pulling unit 50, the operator can separate the pulling unit 50 from the main body portion 20 by carrying out simple work for pulling the pulling unit 50 from the connection section (the insertion hole) 60. In a case where the pulling unit 50 is formed of a biodegradable material, the pulling unit 50 may be caused to indwell the living body in a state of being connected to the main body portion 20.

As described above, the adhesion promotion device 100 according to the present embodiment has the main body portion 20 formed of the biodegradable sheet having the plurality of through-holes 25 and promoting the adhesion of the biological tissues, and the assistance unit 40 provided in the main body portion 20 and assisting the main body portion 20 to hold the biological organ serving as the joint object.

According to the adhesion promotion device 100, the adhesion of the biological tissues of the biological organ can be promoted by pinching the main body portion 20 between the biological organs serving as the joint object. In addition, while the operator performs the medical procedure, the operator uses the assistance unit 40 provided in the main body portion 20 to assist the main body portion 20 to hold the biological organ serving as the joint object. In this manner, it is possible to prevent a possibility that the main body portion 20 may be misaligned from the biological organ or may be distorted and deformed. Therefore, the operator can effectively reduce risk factors of an anastomotic leakage of the biological organs.

In addition, the assistance unit 40 includes the pulling unit 50 pulled to apply the pulling force to the main body portion 20, and the connection section 60 enabling the pulling unit 50 to be connected to the main body portion 20. The operator can more reliably hold the main body portion 20 for the biological organ by applying the pulling force to the main body portion 20 via the pulling unit 50. In addition, the operator can easily connect the pulling unit 50 to the main body portion 20 at any desired timing by providing the connection section 60 in the adhesion promotion device 100. In addition, the operator can freely select whether to use the pulling unit 50 or not during the medical procedure.

In addition, the connection section 60 is configured to include the insertion holes formed in the main body portion 20 and penetrating the main body portion 20 in the thickness direction. Therefore, the operator can connect the pulling unit 50 to the main body portion 20 by carrying out easy work for inserting the pulling unit 50 into the insertion hole.

In addition, in the main body portion 20, the peripheral edge portion 26 of the main body portion 20 has the reinforcement portion 27 having rigidity higher than that of the other portion of the main body portion 20. The connection section (insertion hole) 60 of the assistance unit 40 is disposed in the reinforcement portion 27. Therefore, when the pulling force is applied to the main body portion 20 via the connection section 60 and the pulling unit 50 inserted into the connection section 60, it is possible to prevent the main body portion 20 from being damaged due to stress concentration in the vicinity of the connection section 60.

In addition, the main body portion 20 has a planar shape which is rotationally symmetric. At least one set of the assistance units 40 is provided to form a pair at facing locations of the main body portion 20. Therefore, since the operator can apply the pulling force to two locations facing the main body portion 20, the operator can more stably hold the main body portion 20 for the biological organ.

In addition, the main body portion 20 has the hole portion 30 having the hole diameter formed to be larger than that of the through-hole 25. Therefore, when the adhesion promotion device 100 is used for the large intestine anastomosis, the adhesion promotion device 100 can be easily mounted on the anastomosis device 500 by using the hole portion 30.

Next, modification examples of the first embodiment described above and other embodiments will be described. In the following description, detailed description of the configurations already described will be omitted. In addition, contents not particularly described can be substantially the same as those in the first embodiment.

### (Modification Example of First Embodiment)

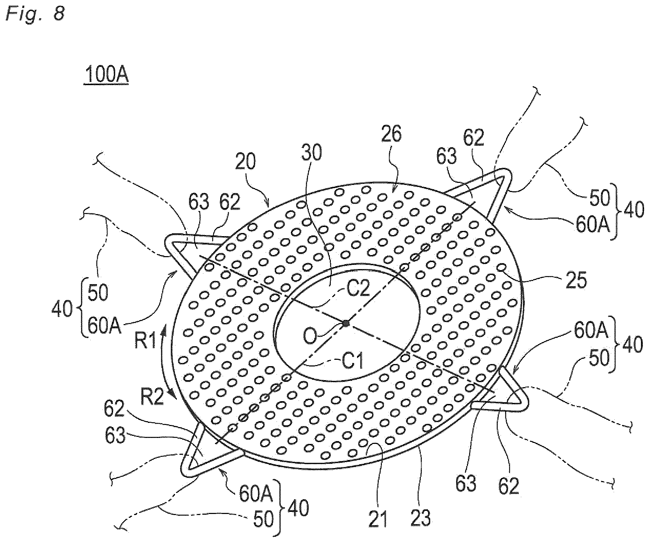

Fig. 8 is a view illustrating an adhesion promotion device 100A according to a modification example.

In the adhesion promotion device 100A according to the modification example, the connection section is configured to include a hook portion 60A including a space portion 63 formed outward of the peripheral edge portion 26 of the main body portion 20 and into which the pulling unit 50 can be inserted.

The hook portion 60A has a frame-shaped member 62 attached to the outer peripheral side surface of the main body portion 20, and the space portion 63 partitioned by the frame-shaped member 62. The operator can connect the pulling unit 50 to the main body portion 20 by inserting the pulling unit 50 into the space portion 63. Therefore, as in the case where the connection section 60 (refer to Fig. 1) configured to include the insertion holes is provided in the main body portion 20, the operator can easily connect the pulling unit 50 to the main body portion 20.

The reinforcement portion 27 is not provided in the main body portion 20 of the adhesion promotion device 100A, but the reinforcement portion 27 may be provided. In addition, the insertion hole together with the hook portion 60A can be provided in the adhesion promotion device 100A. In addition, a specific shape or a configuration material of the hook portion 60A is not particularly limited as long as the pulling unit 50 can be inserted.

### (Second Embodiment)

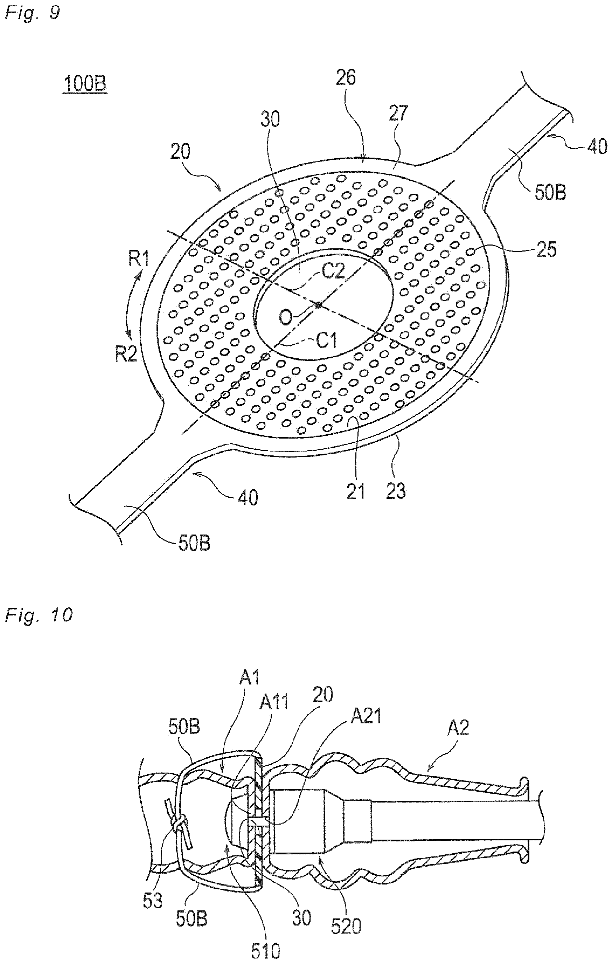

Fig. 9 is a view illustrating an adhesion promotion device 100B according to a second embodiment. Fig. 10 is a view illustrating a procedure example of a medical procedure using the adhesion promotion device 100B.

In the adhesion promotion device 100B according to the second embodiment, a pulling unit 50B is configured to include a member integrally provided in the main body portion 20 and having a predetermined length.

The pulling unit 50B is configured to be integral with the reinforcement portion 27 provided in the peripheral edge portion 26 of the main body portion 20. Two pulling units 50B are provided in the main body portion 20. Each of the pulling units 50B is disposed at a facing location of the main body portion 20.

The pulling unit 50B is configured to include a strip-shaped member having a predetermined width dimension in a direction intersecting with an extending direction of the pulling unit 50B. In addition, a portion of the pulling unit 50B is formed of a flexible material in view of an operation for pulling the pulling unit 50B in parallel with the biological organ (mouth side A1 of the large intestine) or an operation for connecting the pulling unit 50B to the outer periphery of the biological organ (to be described later).

As illustrated in Fig. 10, in the medical procedure using the adhesion promotion device 100B, the operator connects the respective pulling units 50B to each other in a state where the respective pulling units 50B are pulled to one end (side to opposite to a side facing the anal side A2 of the large intestine) of the large intestine. In this manner, the operator can form a knot 53. The operator can maintain a state where the pulling force is applied to the respective pulling units 50B by forming the knot 53 on the outer periphery of the biological organ as described above.

In addition, as illustrated in Fig. 9, the pulling unit 50B is configured to be integral with the reinforcement portion 27 disposed over the entire periphery of the peripheral edge portion 26 of the main body portion 20. Therefore, when the pulling force is applied to the pulling unit 50B, the reinforcement portion 27 can effectively prevent the main body portion 20 from being damaged.

As described above, in the adhesion promotion device 100B according to the present embodiment, the pulling unit 50B is configured to include the member integrally provided in the main body portion 20 and having the predetermined length. Therefore, work for connecting the pulling unit 50 to the main body portion 20 can be omitted. Therefore, the medical procedure using the adhesion promotion device 100B can be more smoothly and progressively performed.

### (Modification Example 1 of Second Embodiment)

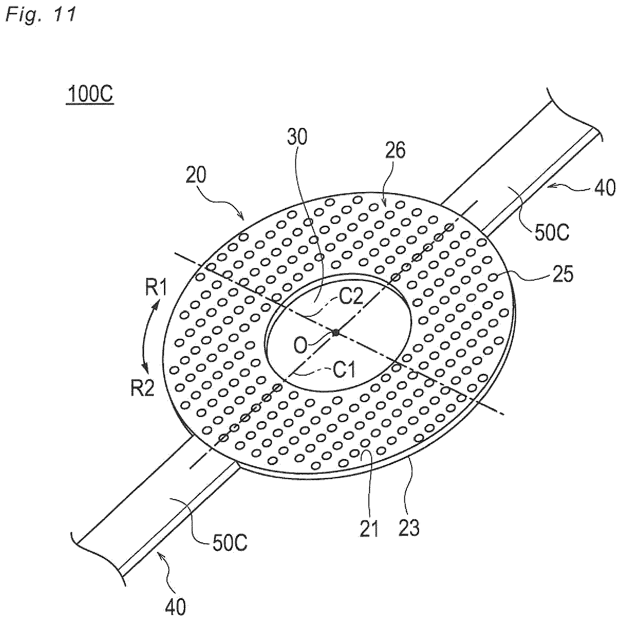

Fig. 11 is a view illustrating an adhesion promotion device 100C according to Modification Example 1 of the second embodiment.

In the adhesion promotion device 100C according to the modification example, a pulling unit 50C is integrally provided in the main body portion 20. However, the reinforcement portion 27 is not provided in the main body portion 20. For example, the pulling unit 50C can be fixed to the main body portion 20 by using a suture, a stapler, or a biocompatible adhesive.

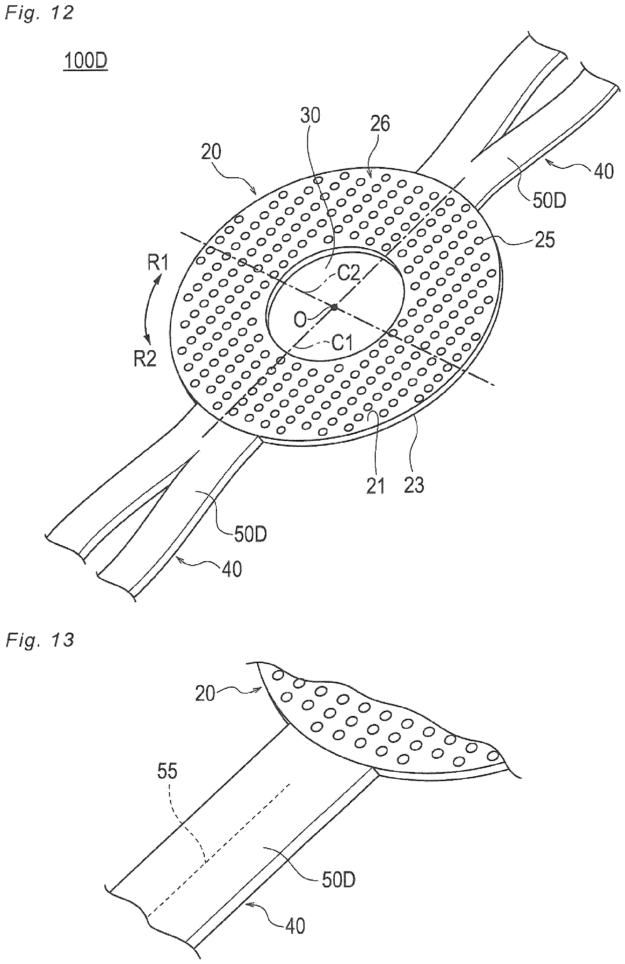

Figs. 12 and 13 are views illustrating an adhesion promotion device 100D according to Modification Example 2 of the second embodiment.

As illustrated in Fig. 13, the pulling unit 50D included in the adhesion promotion device 100D has an easily dividable portion 55 that can divide the pulling unit 50D into two or more pieces along a longitudinal direction.

As illustrated in Fig. 12, the operator can divide the pulling unit 50D into a plurality of divided pieces by using the easily dividable portion 55 during the medical procedure. After dividing the pulling unit 50D, while the operator applies the pulling force to each of the divided pieces, and winds each of the divided pieces of the pulling unit 50D around the outer periphery (for example, the mouth side A1 of the large intestine) of the biological organ. In this manner, the main body portion 20 can be held in the biological organ. Therefore, the operator can stably hold the main body portion 20 in the biological organ without using a medical instrument such as intestinal forceps.

The number, a disposition, or a shape of the easily dividable portions 55 provided in one pulling unit 50D is not particularly limited. In addition, for example, the easily dividable portion 55 can be configured to include a notch formed in a portion of the pulling unit 50D, a portion having a thin wall thickness, or a portion formed of a material which is more easily broken than other portions.

### (Modification Example 3 of Second Embodiment)

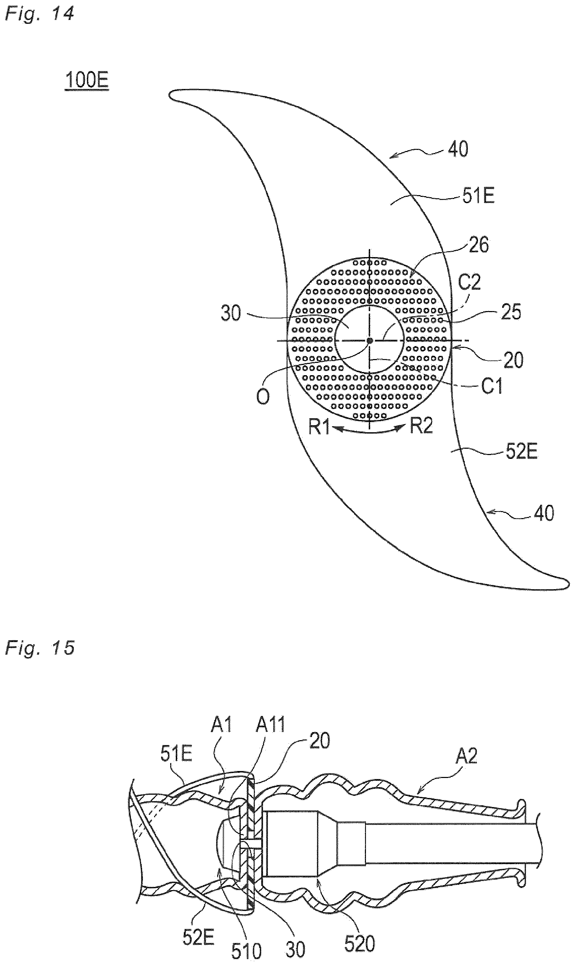

Fig. 14 is a view illustrating an adhesion promotion device 100E according to Modification Example 3 of the second embodiment. Fig. 15 is a view illustrating a procedure example of a medical procedure using the adhesion promotion device 100E.

In the adhesion promotion device 100E, two pulling units 51E and 52E provided in the main body portion 20 extend to be curved outward in a direction separated from the central position O of the main body portion 20.

As illustrated in Fig. 14, the pulling unit 51E is curved while a distal portion side located in the extending direction is gradually narrowed toward one side in the circumferential direction of the main body portion 20. The pulling unit 52E has a symmetrical shape with the pulling unit 51E in a rightward-leftward direction in Fig. 14 with reference to the central position O of the main body portion 20. In the illustrated example, the reinforcement portion 27 is not provided in the peripheral edge portion 26 of the main body portion 20, but the reinforcement portion 27 may be provided in the main body portion 20. In addition, a specific shape of the respective pulling units 51E and 52E is not particularly limited.

As illustrated in Fig. 15, in the medical procedure using the adhesion promotion device 10E, the operator, for example, the operator winds the respective pulling units 51E and 52E around the outer periphery of the biological organ (for example, the mouth side A1 of the large intestine) in a state where the respective pulling units 51E and 52E are pulled to one end (side opposite to a side facing the anal side A2 of the large intestine) of the large intestine. In this manner, the main body portion 20 can be held for the biological organ. Therefore, the operator can stably hold the main body portion 20 in the biological organ without using a medical instrument such as intestinal forceps. In addition, the respective pulling units 51E and 52E extend to be curved outward in the direction separated from the central position O of the main body portion 20. Accordingly, the respective pulling units 51E and 52E can be easily wound around the biological organ, and the respective pulling units 51E and 52E can be more reliably prevented from being unwound and separated from the biological organ.

### (Modification Example 4 of Second Embodiment)

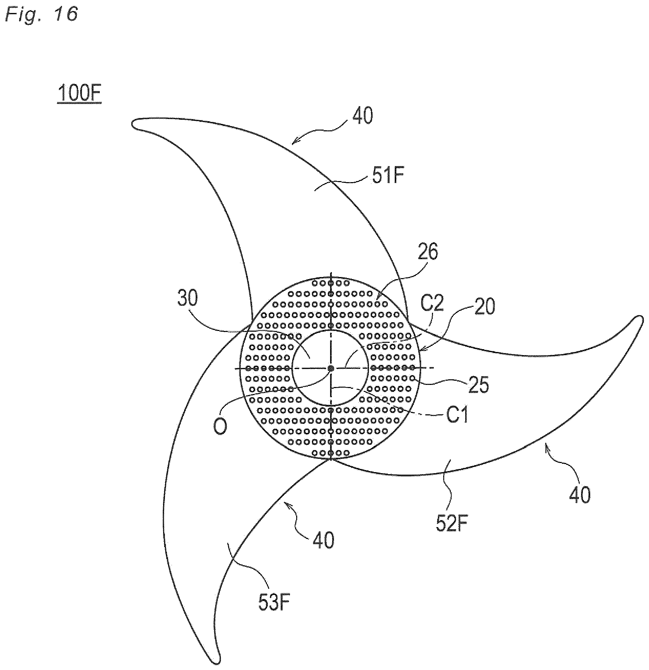

Fig. 16 is a view illustrating an adhesion promotion device 100F according to Modification Example 4 of the second embodiment. As illustrated in Fig. 16, in the adhesion promotion device 100F, three pulling units 51F, 52E, and 53F having a curved shape are disposed at different positions in the circumferential direction of the main body portion 20. Holding power when the pulling unit 50F is wound around the biological organ can be improved by increasing the number of the pulling units 51F, 52F, and 53F as illustrated in the present modification example.

### (Other Embodiments)

In the respective embodiments and the modification examples as described above, an example has been described in which the assistance unit 40 (at least one of the pulling unit and the connection section) is provided in at least a portion of the peripheral edge portion 26 of the main body portion 20. However, a position for providing the assistance unit 40 in the main body portion 20 is not limited only to the peripheral edge portion 26. For example, in a case where the hole portion 30 is provided in the main body portion 20, the assistance unit 40 can be provided in the periphery (innermost peripheral portion) of the hole portion 30. In addition, in a case where the assistance unit 40 is disposed in the periphery of the hole portion 30, the reinforcement portion 27 can be provided to surround the hole portion 30, and the assistance unit 40 can be provided in the reinforcement portion 27.

### (Assistance Device 200)

Next, an assistance device 200 according to the present invention will be described.

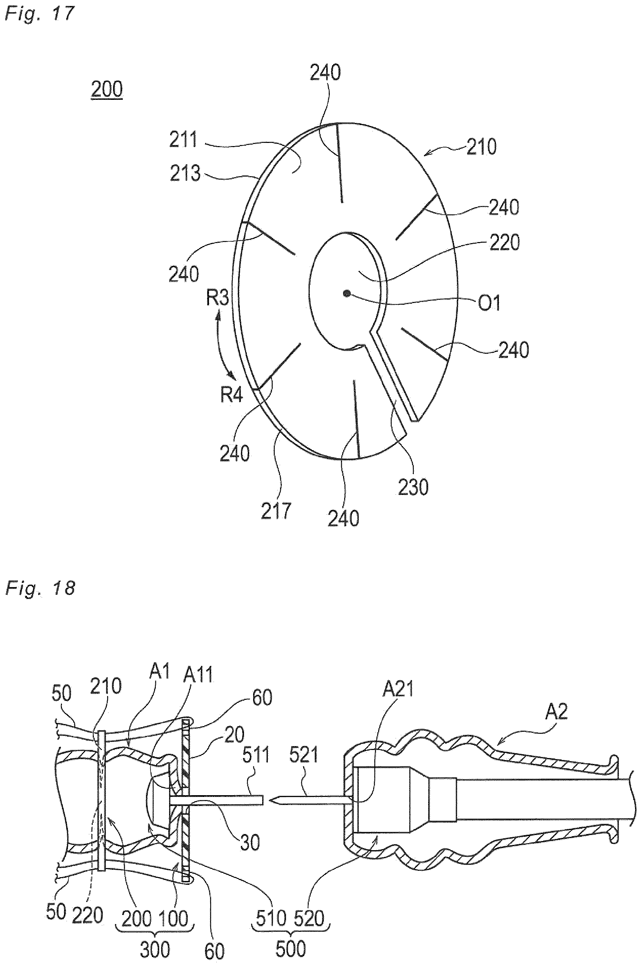

Fig. 17 is a perspective view illustrating the assistance device 200. Fig. 18 is a cross-sectional view schematically illustrating an example of a treatment procedure using the assistance device 200.

As illustrated in Fig. 18, the assistance device 200 and the adhesion promotion device 100 configure a medical device 300 which is preferably used for predetermined anastomosis.

As illustrated in Fig. 17, the assistance device 200 has a mounting portion 210 that can be mounted on the outer periphery of the biological organ (for example, the mouth side A1 of the large intestine), an insertion portion 220 formed in the mounting portion 210 and into which the biological organ is inserted, a communicating section 230 formed in the mounting portion 210 and causing an outer peripheral side surface 217 of the mounting portion 210 and the insertion portion 220 to communicate with each other, and an attachment section 240 formed in the mounting portion 210 and to which a member (for example, the pulling unit 50) for connecting the mounting portion 210 and the main body portion 20 of the adhesion promotion device 100 to each other can be attached.

As illustrated in Fig. 18, the assistance device 200 is used to maintain the pulling force applied to the main body portion 20, when the adhesion promotion device 100 is held for the biological organ. Specifically, in a state where the mounting portion 210 of the assistance device 200 is mounted on the biological organ, the pulling unit 50 is connected to the mounting portion 210. In this manner, the assistance device 200 holds the adhesion promotion device 100 for the biological organ. Therefore, since the operator uses the assistance device 200, the operator can hold the main body portion 20 in the biological organ without using a medical instrument such as intestinal forceps.

As illustrated in Fig. 17, the mounting portion 210 has a substantially circular planar shape. The insertion portion 220 is formed by a substantially circular through-hole formed in a predetermined range including the central position of the mounting portion 210. The communicating section 230 is configured to include a gap portion having a predetermined width.

When the mounting portion 210 is mounted on the biological organ, the operator guides the biological organ into the insertion portion 220 through the communicating section 230. In addition, the biological organ guided to the insertion portion 220 via the communicating section 230 is restrained in the periphery of the insertion portion 220, and is deformed to a partially bent state. The mounting portion 210 is mounted along the outer peripheral portion of the biological organ in a bent state.

The attachment section 240 is configured to include a slit (notch) penetrating between a front surface 211 and a rear surface 213 of the mounting portion 210. A plurality of the attachment sections 240 are provided in the mounting portion 210. The respective attachment sections 240 are disposed at different locations in the circumferential direction of the mounting portion 210.

The respective attachment sections 240 extend from the outer peripheral side surface 217 of the mounting portion 210 toward a central position O1 of the mounting portion 210. One end portion of the respective attachment sections 240 reaches the outer peripheral side surface 217 of the mounting portion 210. In addition, the other end portion on a side opposite to one end portion of the respective attachment sections 240 does not extend to the insertion portion 220, and is located on the outer peripheral side surface 217 of the mounting portion 210 from the insertion portion 220.

It is preferable that the mounting portion 210 is formed of a material harder than that of the main body portion 20 of the adhesion promotion device 100 so that the mounting portion 210 is not distorted and deformed when the mounting portion 210 is mounted on the biological organ. For example, the mounting portion 210 can be formed of a hard resin, silicone, or PTFE. An inner peripheral surface of the insertion portion 220 may be coated to increase friction generated between the insertion portion 220 and the biological organ, and a non-slip member for increasing the friction may be disposed on the inner peripheral surface of the insertion portion 220.

As illustrated in Fig. 18, when the assistance device 200 is used, the operator can hold the main body portion 20 for the assistance device 200 by hooking the pulling unit 50 on the attachment section 240. In the assistance device 200, the attachment section 240 extends from the outer peripheral side surface 217 of the mounting portion 210 to the central position of the mounting portion 210. Accordingly, the operator can easily hook the pulling unit 50 on the attachment section 240.

The operator attaches the pulling unit 50 to the attachment section 240. In this manner, the main body portion 20 can be held for the biological organ without gripping or pulling the pulling unit 50 with the operator's own fingers. Therefore, the assistance device 200 can reduce a workload of the operator when the main body portion 20 is held for the biological organ.

The adhesion promotion device used together with the assistance device 200 may adopt any one of the above-described respective embodiments and the modification examples. In addition, a specific shape, a structure, and a size of each portion of the assistance device 200 are not limited to those described in the illustrated example, and can be changed as appropriate. In addition, a member other than the pulling unit may be used as a member used for connecting the assistance device 200 and the adhesion promotion device to each other.

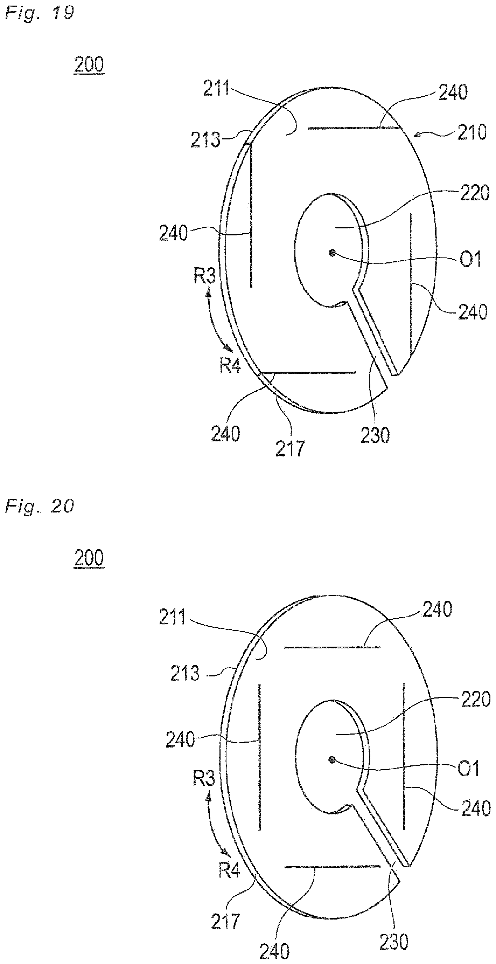

For example, as illustrated in Fig. 19, the attachment section 240 formed in the assistance device 200 may not extend to the central position O1 of the mounting portion 210, and may have a shape substantially linearly extending from the outer peripheral side surface 217 of the mounting portion 210. In addition, for example, as illustrated in Fig. 20, the attachment section 240 formed in the assistance device 200 may not face the central position O1 of the mounting portion 210, and may have a shape which does not extend to the outer peripheral side surface 217 of the mounting portion 210. In a case where the attachment section 240 illustrated in Fig. 19 or Fig. 20 is formed, when the pulling unit 50 is pulled from the adhesion promotion device 100 side to the assistance device 200 side, the assistance device 200 can prevent the pulling unit 50 from interfering with or being hooked on the first engagement instrument 510 (refer to Fig. 18) of the anastomosis device 500 disposed between the assistance device 200 and the main body portion 20.

The attachment section 240 may have a structure other than the slit (notch) . For example, the attachment section 240 may be a hole portion formed in the mounting portion 210.

Hitherto, the adhesion promotion device and the medical device according to the present invention have been described through the embodiments. However, the present invention is not limited only to the contents described in the embodiments, and can be appropriately modified, based on the appended claims.

For example, the biological organ serving as the joint object, the joint target site, and the specific medical procedure are not limited to those described in the embodiments. In addition, the material, the size, the shape, and the specific structure of the medical instrument are not particularly limited, as long as the medical instrument has a function of promoting the adhesion of the biological tissues of the joint target site by using the main body portion included in the adhesion promotion device.

In addition, the respective embodiments and the respective structures described in the modification examples can be combined with each other in any desired way for one adhesion promotion device, as long as advantageous effects of the invention can be achieved. The same applies to the assistance device.

This application is based on Japanese Patent Application No. 2018-181774 filed on September 27, 2018, the disclosure content of which is incorporated by reference in its entirety.

### Reference Signs List

- 10, 10A, 10B, 10C, 10D, 10E: adhesion promotion device
- 20: main body portion
- 25: through-hole
- 26: peripheral edge portion of main body portion
- 27: reinforcement portion
- 30: hole portion
- 40: assistance unit
- 50, 50B, 50C, 50D, 51E, 52E, 51F, 52F, 53F: pulling unit
- 53: knot
- 55: easily dividable portion
- 60: connection section (insertion hole)
- 60A: hook portion (connection section)
- 62: frame-shaped member
- 63: space portion
- 200: assistance device
- 210: mounting portion
- 217: outer peripheral side surface of mounting portion
- 220: insertion portion
- 230: communicating section
- 240: attachment section
- 300: medical device
- 500: anastomosis device
- 510: first engagement instrument
- 520: second engagement instrument
- O: central position of main body portion
- O1: central position of mounting portion
- A1: mouth side of large intestine (biological organ)
- A2: anal side of large intestine (biological organ)

## Claims

1. An adhesion promotion device comprising:
a main body portion formed of a biodegradable sheet having a plurality of through-holes and promoting adhesion of biological tissues; and
an assistance unit provided in the main body portion and assisting the main body portion to hold a biological organ serving as a joint object.

2. The adhesion promotion device according to Claim 1,
wherein the assistance unit includes at least one of a pulling unit pulled to apply a pulling force to the main body portion and a connection section enabling the pulling unit to be connected to the main body portion.

3. The adhesion promotion device according to Claim 2,
wherein the connection section has at least one of an insertion hole formed in the main body portion and penetrating the main body portion in a thickness direction, and a hook portion including a space portion formed outward of a peripheral edge portion of the main body portion and into which the pulling unit is insertable.

4. The adhesion promotion device according to Claim 2 or 3,
wherein in the main body portion, the peripheral edge portion of the main body portion has a reinforcement portion having rigidity higher than that of other portions of the main body portion, and
at least a portion of the assistance unit is disposed in the reinforcement portion.

5. The adhesion promotion device according to any one of Claims 2 to 4,
wherein the pulling unit is formed of a member integrally provided in the main body portion and having a predetermined length.

6. The adhesion promotion device according to Claim 5,
wherein the pulling unit has an easily dividable portion that enables the pulling unit to be divided into two or more along a longitudinal direction.

7. The adhesion promotion device according to Claim 5 or 6, wherein the pulling unit extends to be curved outward so that the pulling unit is separated from a central position of the main body portion.

8. The adhesion promotion device according to any one of Claims 1 to 7,
wherein the main body portion has a planar shape which is rotationally symmetric, and
at least one set of the assistance units is provided to form a pair at facing locations of the main body portion.

9. The adhesion promotion device according to any one of Claims 1 to 8,
wherein the main body portion further has a hole portion formed to have a hole diameter larger than that of the through-hole.

10. A medical device comprising:
the adhesion promotion device according to any one of Claims 1 to 9; and
an assistance device having a mounting portion mountable along an outer periphery of the biological organ, and connected to the main body portion in a state of being mounted on the biological organ so that the adhesion promotion device is held by the main body portion.

11. The medical device according to Claim 10,
wherein the assistance device has an insertion portion formed in the mounting portion and into which the biological organ is inserted, a communicating section formed in the mounting portion and causing an outer peripheral side surface of the mounting portion and the insertion portion to communicate with each other, and an attachment section formed in the mounting portion capable of attaching a member for connecting the mounting portion and the main body portion to each other.
